# EUROPÄISCHE PATENTSCHRIFT

(11) **EP 3 077 498 B1**
(45) Veröffentlichungstag und Bekanntmachung des Hinweises auf die Patenterteilung: **27.02.2019**
(21) Anmeldenummer: 14809820.5
(22) Anmeldetag: 05.12.2014
(51) Int. Cl.: A23N 1/00, C02F 11/00, C12M 1/00, C12M 1/42, C12M 1/107

(54) **VORRICHTUNG ZUR ELEKTRISCHEN DESINTEGRATION MIT MEHREREN WIRKZONEN**
DEVICE FOR ELECTRICAL DISINTEGRATION WITH SEVERAL ACTIVE ZONES
DISPOSITIF DE DÉSINTÉGRATION ÉLECTRIQUE COMPRENANT PLUSIEURS ZONES D'ACTION

(30) Priorität: 06.12.2013 DE 202013009847 U
(43) Veröffentlichungstag der Anmeldung: 12.10.2016
(73) Patentinhaber: Hugo Vogelsang Maschinenbau GmbH, 49632 Essen (DE)
(72) Erfinder: HOLLAH, Clemens, 49632 Essen (DE)
(74) Vertreter: Eisenführ Speiser
(86) Internationale Anmeldenummer: PCT/EP2014/076756
(87) Internationale Veröffentlichungsnummer: WO 2015/082700

(56) Entgegenhaltungen:
- DE-A1-102005 029 414
- US-A- 4 970 154
- US-A- 5 690 978

## Beschreibung

Die vorliegende Erfindung betrifft eine Vorrichtung zur elektrischen Desintegration von Zellverbänden, mit einer Elektrodeneinheit, welche einen Elektrodenkopf und einen Elektrodenkörper aufweist, und einer Kammer, die einen Einlass zur Aufnahme von Zellverbände enthaltendem Fluid und einen Auslass zur Abgabe des Fluids aufweist, wobei die Kammer eine Wand aufweist und der Elektrodenkörper innerhalb der Kammer und von der Wand elektrisch isoliert angeordnet ist. Die Erfindung betrifft ferner eine Vorrichtung zur elektrischen Desintegration von Zellverbänden, mit einer Kammer, die einen Einlass zur Aufnahme von Zellverbände enthaltendem Fluid und einen Auslass zur Abgabe des Fluids aufweist, wobei die Kammer eine Wand aufweist und dazu angepasst ist, einen Elektrodenkörper derart innerhalb der Kammer aufzunehmen, dass dieser von der Wand elektrisch isoliert angeordnet ist.

Eine derartige Vorrichtung ist aus DE 20 2011 004 177 U1 der hiesigen Anmelderin bekannt. Solche Vorrichtungen werden in unterschiedlichen Bereichen eingesetzt, vornehmlich zur Behandlung von Fluidgemischen mit organischer Materie, insbesondere Zellen und/oder Zellverbände enthaltend, in Biogasanlagen und Kläranlagen. Ziel ist es, mittels der Desintegration der Zellverbände beispielsweise bei Biogasanlagen die Erzeugung von Biogas zu begünstigen, weil durch das sogenannte Cracken der Zellverbände die Reaktion von Ausgangsstoffen zu Faulgas begünstigt wird. Unter dem Begriff der Desintegration wird allgemein die Zerkleinerung von Zellen oder Zellverbänden unter Einwirkung äußerer Kräfte verstanden.

Andere bekannte Desintegrationsmethoden sind die thermische Desintegration, die Ultraschalldesintegration, chemische Desintegration und die mechanische Desintegration.

Die elektrische Desintegration beruht auf dem Funktionsprinzip, Zellverbände einem elektrischen Feld auszusetzen, welches zwischen zwei Elektroden anliegt. Infolge der Einwirkung des elektrischen Feldes auf die Zellen und Zellverbände kommt es an den Zellmembranen zu Ladungsverschiebungen. Bei den bekannten Anlagen zur elektrischen Desintegration wird hierbei sodann ausgenutzt, dass die Zellen und Zellverbände sich innerhalb der Kammer, in der das elektrische Feld anliegt, bewegen. Durch die Bewegung der Zellen und Zellverbände ändert sich im Bezug auf deren jeweilige Zellmembrane lokal die sie beeinflussende Feldstärke. Durch diese andauernde Änderung wird die Zellmembran und/oder der Zellverband Scherkräften und Vibrationen ausgesetzt, was zu einer Destabilisierung führt. Bei ausreichend starker Anregung wird der Zellverband aufgelockert oder aufgelöst. Bei stärkerer Beeinflussung werden die Zellmembranen kollabiert. Letzteres ist unter dem Begriff der Elektroporation bekannt. Der Effekt dieser Desintegration ist jener, dass die Verfügbarkeit insbesondere von Nährstoffen für fermentierende Bakterien deutlich erhöht wird. Dieser Effekt wird in Biogasanlagen vorteilhaft dazu genutzt, eine gesteigerte Gasausbeute und bessere Nutzung der dort angesetzten Substrate zu erreichen. Anlagen unter Nutzung der elektrischen Desintegration und des Verfahrens zur elektrischen Desintegration selbst sind den alternativen Desintegrationsverfahren im Bezug auf die aufzuwendenden Investitionskosten, die Energieaufnahme und den apparativen Aufwand überlegen.

In der eingangs genannten DE 20 2011 004 177 U1 wird vorgeschlagen die Desintegrationsleistung dadurch zu erhöhen, dass eine Steuereinheit mit der Hochspannungsquelle zusammenwirkt, um das elektrische Feld zu ändern, wobei die Steuereinheit dazu eingerichtet ist, die Spannung zwischen der Elektrode und der Wand zu verändern. Es hat sich herausgestellt, dass die Änderung des elektrischen Feldes zu einer deutlichen Leistungssteigerung bei der Desintegration führt.

Weiterhin offenbart US 5,690,978 eine Vorrichtung zu Sterilisation und Konservierung von pumpbaren Lebensmitteln mittels elektrischer Felder. Die Vorrichtung umfasst wenigstens zwei rohrförmige Elektroden, die die durch einen rohrförmigen isolierenden Abschnitt getrennt sind.

Aus US 4,970,154 ist eine Vorrichtung zur Poration und Fusion von Zellen bekannt. Diese Vorrichtung umfasst in einer Ausführungsform ein Rohr mit ringförmig an diesem angeordneten Elektroden, die jeweils abwechselnd unterschiedlich gepolt angeordnet sind, sodass sich zwischen benachbarten Elektrodenringen ein elektrisches Feld ausbilden kann.

Ferner offenbart DE 10 2005 029 414 A1 eine Messzelle zur Bestimmung des durch Elektroporation bewirkenden Aufschlussgrades biologischer Zellen, die für ein resistiv/kapazitives Messen aufgebaut oder für ein induktives Messen aufgebaut ist. In einem Rohr sind hierzu zwei Messelektroden angeordnet.

Vorrichtungen gemäß dem Stand der Technik sind allerdings in der Regel für Großanlagen angepasst. Dementsprechend ist sowohl Ihre Baugröße als auch ihr Gewicht in der Regel hoch. Aufgabe der vorliegenden Erfindung ist es eine Vorrichtung der eingangs genannten Art anzugeben, bei der auch kleinere Massendurchsätze effizient dem Desintegrationsvorgang unterzogen werden können, die insbesondere eine geringere Baugröße sowie ein geringeres Gewicht aufweist und vorzugsweise bezüglich Einbau und Montage vereinfacht ist. Ferner lag der Erfindung insbesondere die Aufgabe zugrunde, die Vorrichtungen der eingangs bezeichneten Art dahingehend zu verbessern, dass bei gleichbleibender Baugröße eine wirksamere Desintegration erreicht wird.

Die Erfindung löst die ihr zugrundeliegende Aufgabe einer Vorrichtung der eingangs bezeichneten Art, indem die Wand eine Mehrzahl voneinander beabstandeter, elektrisch leitfähiger Abschnitte aufweist, wobei der Elektrodenkörper innerhalb der Kammer und von der Wand elektrisch isoliert angeordnet ist, und die elektrisch leitfähigen Abschnitte derart angeordnet sind, dass mittels Anlegen einer elektrischen Spannung zwischen dem Elektrodenkörper und der Wand eine Mehrzahl an Wirkfeldern erzeugbar ist. Als Wirkfeld wird vorliegend ein Bereich mit einem lonenstrom verstanden, mittels dem die Zellverbände desintegriert werden. Die voneinander beabstandeten elektrisch leitfähigen Abschnitte dienen als zweite Elektrode (Anode) und leiten das mittels Anlegen der Spannung an den Elektrodenkörper erzeugte elektrische Feld ab, sodass sich ein lonenstrom zwischen dem Elektrodenkörper und den elektrisch leitfähigen Abschnitten der Wand ausbildet.

Die Erfindung beruht auf der Erkenntnis, dass eine kompaktere Bauweise möglich ist, wenn in einer Kammer mittels einer Elektrode mehrere Wirkfelder aufgebaut werden. Die mehreren Wirkfelder bilden somit über die Länge der Kammer in Durchflussrichtung hintereinander angeordnete Wirkzonen, die das der Desintegration zu unterziehende Fluid nacheinander durchströmt. Die Mehrzahl der Wirkfelder entspricht der Mehrzahl der elektrisch leitfähigen Abschnitte. Dies wird dadurch realisiert, dass die Wand der Kammer nur abschnittsweise elektrisch leitfähig ist. Dadurch ist es möglich, in diesen leitfähigen Abschnitten das elektrische Feld abzuleiten und so einen lonenstrom zu erzeugen. Dadurch erfährt das Fluid, welches durch die Kammer strömt, eine auf die Kammerlänge bezogen häufigere Feldstärkenänderung, wodurch die Zellverbände und/oder Zellmembrane destabilisiert und so desintegriert werden. Erfindungsgemäß sind die elektrisch leitfähigen Abschnitte jeweils abwechselnd mit elektrisch isolierenden Abschnitten entlang einer Zentralachse der Kammer angeordnet. In den elektrisch isolierenden Abschnitten kann demnach keine Ableitung des elektrischen Feldes erfolgen und es bildet sich kein lonenstrom zwischen der Elektrode und dem elektrisch isolierenden Abschnitten der Wand aus. Die Zentralachse ist vorzugsweise die Längsachse der Kammer. Vorzugsweise erstreckt sich der Elektrodenkörper entlang dieser Zentralachse. Dies ermöglicht eine einfache Anordnung, wodurch zugleich eine effiziente Destabilisierung von durch die Kammer strömenden Zellverbänden ermöglicht wird.

Vorzugsweise erstrecken sich die Abschnitte jeweils ringförmig um die Kammer herum. Demnach erstrecken sich sowohl die isolierenden Abschnitte, als auch die elektrisch leitfähigen Abschnitte jeweils ringförmig um die Kammer herum. Ringförmig umfasst hier jede Art von Anordnung, bei der ein Abschnitt eine geschlossene Anordnung um die Zentralachse herum bildet. Der Begriff "ringförmig" umfasst zylindrische und nicht zylindrische Querschnittsformen, wobei die zylindrische Ringform besonders bevorzugt wird. Axiale Enden der ringförmigen Abschnitte können jegliche Kontur aufweisen, beispielsweise gerade, geschwungen, schräg bezogen auf eine Zentralachse. Eine besonders einfache Fertigung ergibt sich bei zylindrischen Abschnitten mit plan (senkrecht zur Zylinderachse) endenden Stirnseiten. Je nach Anwendungsfall kann aber auch eine andere Form bevorzugt sein. Indem die Abschnitte jeweils ringförmig geschlossen um die Zentralachse herum ausgebildet sind, wird sichergestellt, dass das elektrische Feld innerhalb der Kammer an diesen Stellen sicher abgeleitet wird und sich ein lonenstrom ausbildet. Es soll verstanden werden, dass die Elektrode zwar ein elektrisches Feld entlang ihrer gesamten Länge aufbauen kann, dieses jedoch nur von den elektrisch leitfähigen Abschnitten der Wand abgeleitet wird und so nur in diesen Abschnitten einen lonenstrom zwischen Elektrode und Wand generiert wird.

In einer besonders bevorzugten Ausführungsform wird die Wand der Kammer von einer inneren Oberfläche eines Rohres gebildet. Das Rohr umschließt demnach die Kammer und das Zellverbände enthaltende Fluid strömt innerhalb des Rohres. Besonders bevorzugt ist das Rohr als zylindrisches Rohr ausgebildet. Dadurch wird einerseits die Herstellung vereinfacht, andererseits kann in einem zylindrischen Rohr das Fluid leichter strömen.

Dabei ist es bevorzugt, dass das Rohr aus ringförmigen Abschnitten aus jeweils elektrisch leitfähigem und/oder elektrisch isolierendem Material gebildet ist. Demnach weist nicht nur die Wand der Kammer die elektrisch leitfähigen und elektrisch isolierenden Abschnitte auf, sondern das Rohr ist insgesamt jeweils abschnittsweise aus elektrisch leitfähigem und elektrisch isolierendem Material gebildet, wodurch auch die Wand abschnittsweise korrespondierend ausgebildet ist.

Gemäß einer bevorzugten Weiterbildung ist das Rohr aus einem dünnwandigen elektrisch isolierenden Material gebildet, wobei um das Rohr mehrere Manschetten aus elektrisch leitendem Material angeordnet sind, die die elektrisch leitenden Abschnitte der Wand bilden. Gemäß dieser bevorzugten Ausführungsform ist zwar die unmittelbar von dem Zellverbände enthaltenden Fluid beströmte Wand aus dem elektrisch isolierenden Material, welches allerdings dünnwandig ausgebildet ist. Das dünnwandige Rohr wird von Manschetten aus elektrisch leitfähigem Material umgeben. Da das elektrische Feld, welches innerhalb der Kammer mittels der Elektrode aufgebaut wird, verhältnismäßig stark ist, kann dieses das dünnwandige isolierende Material des Rohres durchdringen und von dem elektrisch leitfähigen Material der Manschetten abgeleitet werden. Demnach sind die von den Manschetten umgebenen Abschnitte elektrisch leitfähige Abschnitte. Dadurch wird an diesen Stellen ein lonenstrom zwischen der Elektrode und den Manschetten erzeugt. Dadurch wird eine einfache Montage erreicht. Alternativ können auch Manschetten innenliegend in dem Rohr eingebracht sein, oder mittels Spritzgießen integriert werden.

Vorzugsweise sind die elektrisch leitenden Abschnitte jeweils von zwei elektrisch isolierenden Abschnitten axial eingeschlossen. Dadurch wird eine besonders effektive Ableitung des elektrischen Feldes ermöglicht und eine Reduktion des lonenstroms in den elektrisch isolierten Bereichen erzielt. Die elektrisch leitfähigen Abschnitte sind demnach nicht untereinander verbunden, sondern stets durch elektrisch isolierende Abschnitte separiert.

In einer bevorzugten Weiterbildung sind wenigstens drei, vier oder fünf elektrisch leitfähige Abschnitte vorgesehen. Hierdurch werden die Länge der Kammer und die Länge des Elektrodenkörpers effektiv ausgenutzt. Bevorzugt ist ferner, dass wenigstens sechs, sieben oder acht elektrisch leitfähige Abschnitte für eine Kammer vorgesehen sind.

Gemäß einer weiteren bevorzugten Ausführungsform ist zwischen zwei benachbarten elektrisch leitfähigen Abschnitten jeweils ein Mindestabstand vorgesehen, welcher wenigstens 150 mm, 200 mm, 250 mm, 300 mm oder 350 mm beträgt. Hierdurch wird sichergestellt, dass das elektrische Feld in den jeweiligen elektrisch leitfähigen Abschnitten abgeleitet wird und nicht zum nächsten elektrisch leitfähigen Abschnitt "überspringt". Dadurch wird die Ausbildung mehreren einzelner Wirkfelder vorteilhaft weitergebildet. Je nach Stärke der verwendeten Elektrode sowie Durchmesser der Kammer können auch andere Werte bevorzugt sein. Jedenfalls hat es sich in der Praxis gezeigt, dass ein Abstand von 350 mm ausreichend ist, um eine effektive Unterbrechung der Mehrzahl der Wirkfelder zu erzielen.

Weiterhin ist bevorzugt, dass die elektrisch leitfähigen ringförmigen Abschnitte eine axiale Ausdehnung von mindestens 100 mm, 150 mm, 200 mm oder 250 mm haben. Diese axialen Ausdehnungen führen alle zu einer effektiven Reduktion oder einem vollständigen Abbaus des elektrischen Feldes.

Besonders bevorzugt sind die elektrisch isolierenden Abschnitte aus einem Kunststoff, insbesondere einem Thermoplast, vorzugsweise ausgewählt aus der Liste bestehend aus: POM (Polyoxymethylen), PE (Polyethylen), PP (Polypropylen), PA (Polyamid), PET (Polyethylenterephtalat), PBT (Polybutylenterephtalat), ABS (Acrylnitril-Butadien-Styrol), PMMA (Polymethylmethacrylat), PS (Polystyrol), PVC (Polyvinylchlorid), PC (Polycarbonat), SAN (Styrol-Acrylnitril-Copolymer), PPE (Polyphenylenether), oder aus einem Duroplast, oder aus einem Gemisch aus mehreren der vorstehenden Stoffe ausgebildet. Dies führt zu einer kostengünstigen Herstellung der Vorrichtung und reduziert das Gewicht. Die Auswahl von PVC wird besonders bevorzugt. Ferner kann auch ein anderes elektrisch nicht leitendes Material wie etwa GFK verwendet werden.

Gemäß einer weiteren besonders bevorzugten Ausführungsform sind die elektrisch leitfähigen Abschnitte geerdet. Vorzugsweise weist jeder elektrisch leitfähige Abschnitt eine separate Erdung auf. Dazu ist vorzugsweise an jedem elektrisch leitfähigen Abschnitt eine Erdungsleitung oder ein Erdungskabel vorgesehen, welches an Masse angeschlossen ist. Vorzugsweise sind sämtliche Erdungsleitungen oder Erdungskabel gemeinsam an einem Erdungsbolzen geführt. Hierdurch wird sichergestellt, dass das elektrische Feld an elektrisch leitfähigen Abschnitten effektiv abgeleitet wird und sich ein lonenstrom ausbildet.

In einer bevorzugten Weiterbildung erstreckt sich der Elektrodenkörper im Wesentlichen durch die gesamte Kammer hindurch. Hierdurch wird eine kompakte Anordnung erreicht und der Elektrodenkörper erstreckt sich durch sämtliche Abschnitte, sowohl die elektrisch leitfähigen Abschnitte, als auch die elektrisch isolierenden Abschnitte hindurch. Vorzugsweise ist dazu in der Kammer wenigstens eine Lagerung für Elektrodenkörper vorgesehen. Demnach wird auch das elektrische Feld in der gesamten Kammer aufgebaut, jedoch nur von den elektrisch leitfähigen Abschnitten der Wand abgeleitet, sodass in diesen Bereichen Wirkfelder und so Wirkzonen ausgebildet werden. In den elektrisch isolierenden Abschnitten ist zwar auch ein elektrisches Feld vorhanden, dieses ist aber aufgrund der fehlenden zweiten Elektrode gering und wird nicht als Wirkfeld bezeichnet.

Gemäß einer weiteren bevorzugten Ausführungsform weist die Vorrichtung ferner ein Gehäuse auf, in welchem die Kammer angeordnet ist und welches räumlich von der Wand beabstandet und elektrisch isoliert ist. Vorzugsweise ist das Gehäuse auch von einem Rohr, welches die Wand der Kammer definiert und bildet, beabstandet und isoliert. Das Rohr verläuft vorzugsweise innerhalb des Gehäuses, jedoch so, dass sowohl der Einlass als auch der Auslass der Kammer von außerhalb des Gehäuses zugänglich sind.

Vorzugsweise ist zwischen der Wand der Kammer und dem Gehäuse jeweils ein Mindestabstand vorgesehen, der vorzugsweise 100 mm, 150 mm, 200 mm oder 250 mm beträgt. Dadurch ist gewährleistet, dass die Kammer bzw. die Wand der Kammer stets beabstandet von weiteren in der Nähe der Vorrichtung angeordneten elektrisch leitfähigen Gegenständen angeordnet ist. Elektrisch leitfähige Gegenstände in der Nähe der Kammer können das elektrische Feld innerhalb der Kammer beeinflussen und ableiten. Daher ist es bevorzugt, das Gehäuse beabstandet von der Kammer anzuordnen. Das Gehäuse wirkt so als Abstandhalter für die Kammer. Hierdurch wird die effektive Desintegration der in der Kammer strömenden Zellverbände weiter verbessert.

Besonders bevorzugt weist das Gehäuse ein Isolationsmaterial auf oder besteht aus diesem. Bevorzugt umfasst ein derartiges Isolationsmaterial einen Kunststoff, insbesondere PE und/oder ein Verbundmaterial, insbesondere ein Verbundkunststoff aus Papier- und/oder Glasfasern mit einem Phenol-Formaldehyd-Kunstharz und/oder einem Epoxidharz. Ein Verbundmaterial aus Papier und Phenol-Formaldehyd-Kunstharz ist auch als Hartpapier bekannt und wird beispielsweise in der Leiterplattenindustrie eingesetzt. Ein derartiges Material hat einerseits gute isolierende Eigenschaften, andererseits bietet es eine gewisse Stabilität. Ferner ist ein solches Material weithin bekannt und kostengünstig.

Gemäß einem weiteren Aspekt der Erfindung wird die eingangs genannte Aufgabe durch eine Vorrichtung zur elektrischen Desintegration von Zellverbänden gelöst, wobei die Vorrichtung eine Kammer aufweist, die einen Einlass zur Aufnahme von Zellverbänden enthaltendem Fluid und einen Auslass zur Abgabe des Fluids aufweist, wobei die Kammer eine Wand aufweist und dazu angepasst ist, einen Elektrodenkörper derart innerhalb der Kammer aufzunehmen, dass dieser von der Wand elektrisch isoliert angeordnet ist, wobei die Wand eine Mehrzahl voneinander beabstandeter, elektrisch leitfähiger Abschnitte aufweist, die derart angeordnet sind, dass mittels Anlegen einer elektrischen Spannung zwischen einem innerhalb der Kammer angeordneten Elektrodenkörper und der Wand eine Mehrzahl an Wirkfeldern erzeugbar ist.

Es soll verstanden werden, dass die Vorrichtung zur elektrischen Desintegration gemäß dem ersten Aspekt der Erfindung, die die Elektrode samt Elektrodenkopf und Elektrodenkörper umfasst sowie die Vorrichtung zur elektrischen Desintegration von Zellverbänden gemäß dem zweiten Aspekt der Erfindung, die dazu angepasst ist, einen Elektrodenkörper aufzunehmen, gleiche und ähnliche Aspekte umfassen, wie sie insbesondere in den Unteransprüchen und bezüglich der vorstehend beschriebenen bevorzugten Ausführungsformen zum ersten Aspekt definiert sind. Bezüglich der bevorzugten Ausführungsformen der Vorrichtung zur elektrischen Desintegration gemäß dem zweiten Aspekt der Erfindung sowie deren Vorteile wird auf die weiter oben beschriebenen bevorzugten Ausführungsformen vollumfänglich Bezug genommen.

Die Erfindung wird im Folgenden unter Bezugnahme auf die beigefügten Figuren näher beschrieben. Hierbei zeigen:
- Figur 1: eine räumliche Darstellung der erfindungsgemäßen Vorrichtung zur Desintegration von Zellverbänden,
- Figur 2: eine schematische Darstellung der Vorrichtung zur Desintegration von Zellverbänden,
- Figur 3: eine perspektivische Darstellung der Vorrichtung zur Desintegration mit einem Gehäuse,
- Figur 4: eine Seitenansicht der Vorrichtung aus Figur 3,
- Figur 5: eine Draufsicht auf die Vorrichtung aus Figur 3 und 4,
- Figur 6: eine schematische Teildarstellung des funktionalen Aufbaus einer elektronischen Steuereinheit für eine Elektrodeneinheit
- Figur 7: eine weitere schematische Teildarstellung des funktionalen Aufbaus der Steuerung gemäß Figur 6, und
- Figur 8: eine weitere schematische Teildarstellung des funktionalen Aufbaus der Steuereinrichtung gemäß Figuren 6 und 7.

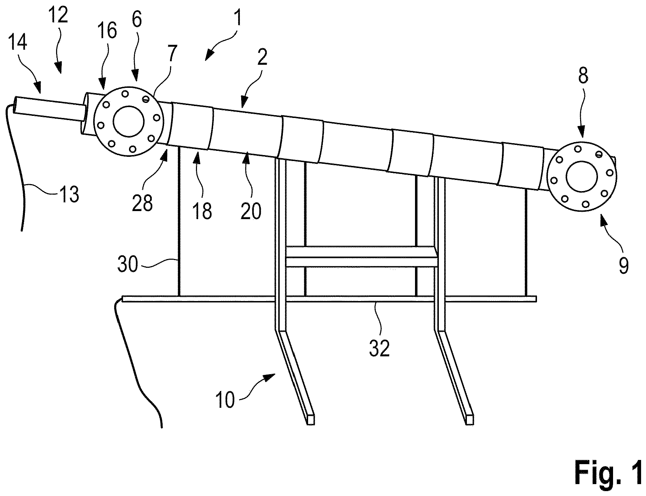

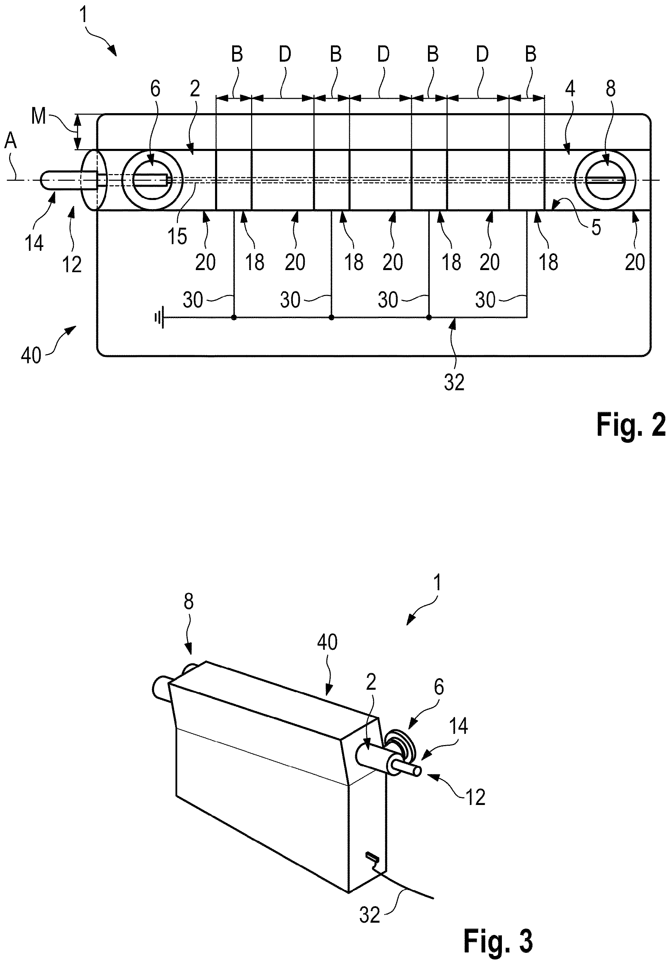

Die in Figur 1 dargestellte Vorrichtung 1 zur elektrischen Desintegration von Zellverbänden weist ein Rohr 2 auf. Das Rohr 2 umschließt eine Kammer 4 (siehe Figur 2) und bildet mit seiner inneren Oberfläche die Wand 5 der Kammer 4. Das Rohr 2 ist im Wesentlichen zylindrisch ausgebildet und weist einen Einlass 6 und einen Auslass 8 auf. An dem Einlass 6 und dem Auslass 8 sind jeweils Flanschanschlüsse 7, 9 angeordnet, so dass die Vorrichtung 1 in ein Rohrleitungssystem einbaubar ist. Gemäß Figur 1 ist das Rohr 1 ferner auf einem Gestell 10 derart gehaltert, dass das Rohr 2 in einem Winkel zur Waagerechten angeordnet ist. Dabei ist der Auslass 8 - bezogen auf eine horizontale Aufstellfläche der Vorrichtung - auf einem niedrigeren Niveau als der Einlass 6 angeordnet. Dadurch wird ein Fließen des Fluids innerhalb der Kammer 4 erleichtert, und sich in der Kammer 4 bildende Blasen können durch den Einlass 6 ausströmen.

Die Vorrichtung 1 weist ferner eine Elektrodeneinheit 12 auf, die einen Elektrodenkopf 14 und einen Elektrodenkörper 15 hat. Die Elektrodeneinheit 12 kann grundsätzlich wie aus DE 20 2011 004 177 U1 bekannt aufgebaut sein. Die Elektrodeneinheit 12 ist mit dem Elektrodenkopf 14 an einer Aufnahme 16 an dem Rohr 2 eingeführt und fluiddicht abgeschlossen. In dem Elektrodenkopf 14 ist die Leistungselektronik der Elektrodeneinheit 12 untergebracht. In Figur 1 ist ferner das Hochspannungskabel 13 gezeigt, mit welchem die Elektrodeneinheit 12 an ein Stromnetz anschließbar ist. Der Elektrodenkörper 15 hingegen ist vollständig innerhalb der Kammer 4 angeordnet und verläuft durch diese vollständig und ist im Bereich des Auslasses 8 an dem Rohr 2 mittels einer nicht gezeigten Lagerung gelagert. Das Rohr 2 weist eine Zentralachse A auf und der Elektrodenkörper 15 erstreckt sich entlang der Zentralachse A.

Wie insbesondere aus Figur 2 ersichtlich, weist die Wand 5 eine Mehrzahl elektrisch leitfähiger Abschnitte, insgesamt mit 18 bezeichnet, auf. Zwischen den elektrisch leitfähigen Abschnitten 18 sind elektrisch isolierende Abschnitte 20 angeordnet. Die elektrisch leitfähigen Abschnitte 18 und die elektrisch isolierenden Abschnitte 20 sind entlang der Kammer 4 jeweils abwechselnd angeordnet. Der Einlass 6 und der Auslass 8 sind in elektrisch isolierenden Abschnitten 20 angeordnet. Die Abschnitte 18, 20 sind jeweils ringförmig um die Zentralachse A herum ausgebildet. Die elektrisch leitfähigen Abschnitte 18 haben jeweils eine axiale Breite B (siehe Figur 2) von 200 mm. Zwischen jeweils zwei benachbarten elektrisch leitfähigen Abschnitten 18 ist ein Abstand D ausgebildet, der gemäß diesem Ausführungsbeispiel 350 mm beträgt.

Wie aus Figur 1 ersichtlich, ist das Rohr 2 als dünnwandiges PVC-Rohr ausgebildet. Die elektrisch leitfähigen Abschnitte 18 (in Figur 1 nur einmal mit Bezugszeichen versehen) sind gemäß diesem Ausführungsbeispiel als Manschetten 28 ausgebildet, die um das Rohr 2 herum gelegt sind. Die Manschetten 28 sind gemäß diesem Ausführungsbeispiel aus VA-Stahl gebildet und demnach elektrisch leitfähig.

Wie insbesondere aus den Figuren 1 und 2 zu erkennen ist, sind die elektrisch leitfähigen Abschnitte 18 geerdet. Dazu ist an jedem elektrisch leitfähigen Abschnitt 18 ein Erdungsband 30 vorgesehen (in Figur 1 nur eins mit Bezugszeichen versehen). Die Erdungsbänder 30 sind gemeinsam an einen Erdungsbolzen angeschlossen, der mit den Erdungsbändern 30 über ein Verbindungsband 32 verbunden ist, welches gemäß diesem Ausführungsbeispiel als Flachstahl ausgebildet ist. Über die Erdung wird erreicht, dass in elektrisch leitfähigen Abschnitten 18 elektrische Spannung abgenommen werden kann und so das elektrische Feld, welches mittels der Elektrode 12 in der Kammer 4 aufgebaut wird abgeleitet wird um einen lonenstrom von der Elektrode 12 zu den jeweiligen elektrisch leitfähigen Abschnitten 18 zu generieren.

Gemäß diesem Ausführungsbeispiel (Figuren 1 und 2) sind vier elektrisch leitfähige Abschnitte 18 in der Vorrichtung 1 vorgesehen, die jeweils mittels des Erdungsbands 30 geerdet sind. Dadurch wird mittels des Elektrodenkörpers 15 ein elektrisches Feld aufgebaut und viermal durch die entsprechenden Erdungen abgeleitet. So werden vier Wirkzonen innerhalb der Kammer 4 erzeugt und zwar in nur einer Kammer mit nur einer Elektrodeneinheit 12.

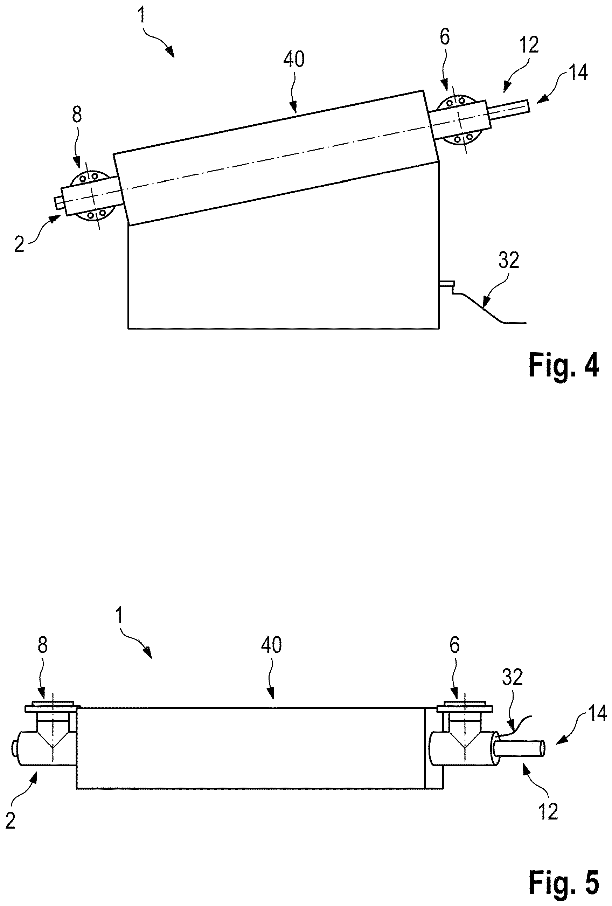

In den Figuren 3 bis 5 ist die Vorrichtung 1 mit einem Gehäuse 40 dargestellt. Das Gehäuse 40 umschließt einen Großteil des Rohres 2, lässt allerdings Einlass 6 und Auslass 8 offen. Das Gehäuse 40 bildet die strukturelle Stütze für das Rohr 2 samt Elektrodeneinheit 12. Zusätzlich kann im Inneren des Gehäuses 40 ein Gestell 10 (vergleiche Figur 1) vorgesehen sein. Wie besonders gut in Figur 3 ersichtlich, ist im unteren Bereich des Gehäuses 40 ein Erdungsband 32 aus diesem herausgeführt. Das Erdungsband 32 wird üblicherweise an einen Erdungsbolzen der dergleichen angeschlossen.

Wie aus Figur 2 zu erkennen ist, weist das Gehäuse 40 einen gewissen Abstand M zur Wand 5 der Kammer 4 auf. Der Abstand M beträgt gemäß diesem Ausführungsbeispiel 200 mm und sorgt dafür, dass elektrisch leitfähige Gegenstände, wie etwa metallische Gegenstände, weitere Rohre oder Maschinenteile in der Umgebung nicht zu nah an das Rohr 2 gelangen, so dass eine Beeinflussung des elektrischen Feldes minimiert wird und ein optimaler Auf- und Abbau der Wirkfelder innerhalb der Kammer 4 gewährleistet wird.

Gemäß diesem Ausführungsbeispiel ist das Gehäuse 40 aus einem Blechkorpus gebildet, der auf seiner inneren Seite und seiner äußeren Seite mit einem isolierenden Material verschalt ist. Gemäß diesem Ausführungsbeispiel ist auf der Innenseite des Gehäuses 40 eine 10 mm dicke Schicht Polyethylen aufgebracht und außen auf dem Gehäuse 40 eine 20 mm dicke Schicht aus einem Verbundmaterial bestehend aus Papier und Phenol-Formaldehyd-Kunstharz. So wird eine gute Stabilität des Gehäuses 40 erreicht und gleichzeitig eine wirksame elektrische Isolierung, die auch das elektrische Feld im Inneren der Kammer 4 abschirmt.

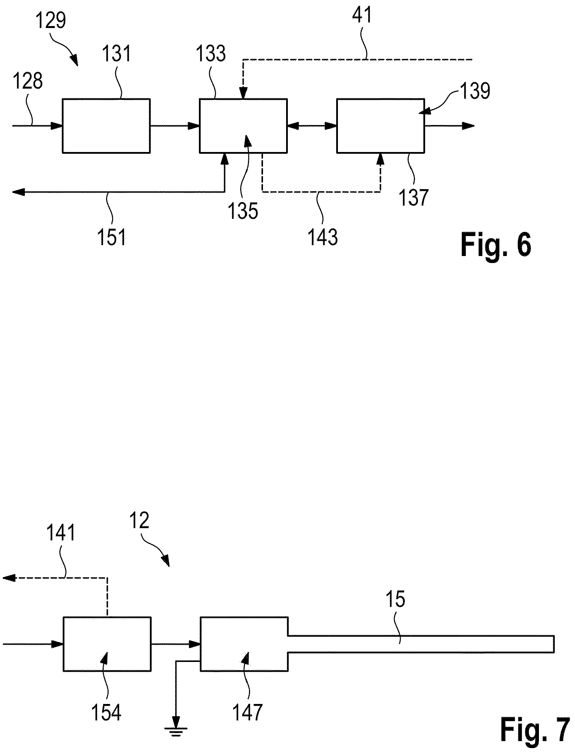

Die Funktion und der Aufbau der Elektrodeneinheit 12 wird nachstehend mit Bezug auf die Figuren 6, 7 und 8 näher erläutert. Die Elektrodeneinheit 12 ist wie bereits erläutert dazu ausgebildet, ein elektrisches Feld zwischen dem Elektrodenkörper 15 und der Wand 5 der Kammer 4 auszubilden. Zum Zwecke der Ansteuerung der Elektrodeneinheit 12 kann die Vorrichtung 1 in einer bevorzugten Ausführungsform eine elektronische Steuereinheit 129 aufweisen. Diese ist in Figur 6 näher erläutert.

Die elektronische Steuereinheit 129 weist ein Netzteil 131 auf, welches einen Spannungseingang 128 umfasst, der beispielsweise zum Anschluss an einen 230V, 50Hz-Wechselspannungsquelle eingerichtet ist. Das Netzteil ist verbunden mit einer Regeleinheit 133, die einen ersten Prozessor 135 enthält. Die Regeleinheit 133 ist zur Bestimmung der Resonanzfrequenz des Systems aus Hochspannungsquelle und Kammer/Elektrodenkörper eingerichtet.

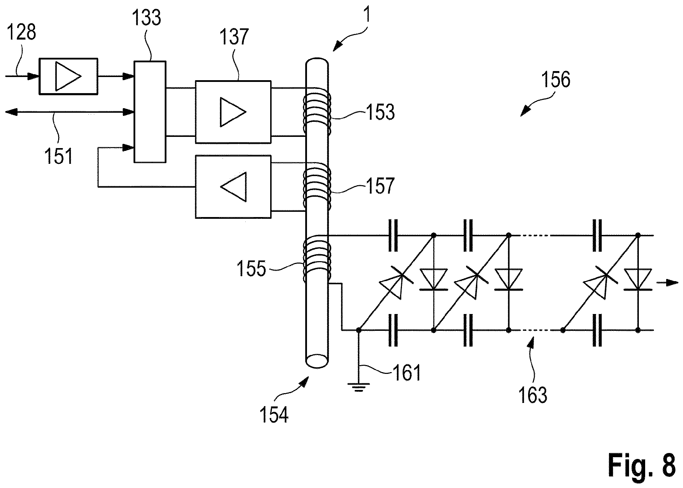

Die Regeleinheit 133 ist mittels Signalleitung 143 mit einer Treibereinheit 137 verbunden, welche einen zweiten Prozessor 139 enthält und zur Ansteuerung einer Spuleinheit 154, welche Teile der Hochspannungsquelle 156 ist (siehe Figur 8) eingerichtet. Eine Datenaustauschleitung 151 ist vorgesehen, um Daten von der Regeleinheit auszulesen, und/oder um diese zu programmieren oder zu steuern.

Im Bereich des Elektrodenkopfes 12 (Figur 7) ist eine Hochspannungskaskade 147 vorgesehen, welche zu einer Vervielfachung der in der Spuleneinheit 154 eingespeisten Spannung führt. Die von der Hochspannungskaskade 147 bereitgestellte Spannung liegt ebenfalls zwischen dem Elektrodenkörper 15 und der Wand 5 an. Die Regeleinheit 133 ist ferner mittels einer Signalleitung 141 mit der Hochspannungsquelle 156 verbunden, um deren Resonanzfrequenz bestimmen zu können. Wie dies vorteilhaft umgesetzt werden kann, ist in Figur 8 veranschaulicht.

Die in Figur 8 gezeigte Spuleinheit 154 weist eine mit der Treibereinheit 137 verbundene Primärspule 153 mit einer ersten Anzahl Windungen auf. Ferner weist die Spuleinheit 154 eine Sekundärspule 155 mit einer zweiten Anzahl Windungen auf, vorzugsweise einem vielfachen an der ersten Anzahl Windungen der Primärspule. Schließlich weist die Spule 154 eine Messspule 157 auf. Alle Spulen 153, 155, 157 sind um den gleichen Spulenkern gespult, beispielsweise einen Ferritkern. Mittels der Primär- und Sekundärspule 153, 155 wird die Eingangsspannung transformiert. Die in der Messspule 157 induzierte Spannung, und vorzugsweise weitere Größen wie etwa der Frequenz, werden von der Regeleinheit gemessen und an diese übertragen.

Die Sekundärspule ist mit einer Vielzahl von Spannungsverdopplern 163 gekoppelt und mittels einer Leitung 161 geerdet. Die vervielfachte Spannung liegt zwischen Elektrodenkörper 15 und Wand 4 an.

Vorzugsweise ist die Elektrodeneinheit 12 so ausgebildet, dass der Elektrodenkopf 14 ein erstes Modul ist und der Elektrodenkörper 15 als zweites Modul ausgebildet ist und die Elektrodeneinheit 12 modular aus den zwei oder mehr koppelbaren Modulen zusammenbaubar ist. Der Elektrodenkopf 14 und Elektrodenkörper 15 können mittels einer Schraubverbindung miteinander verschraubt sein. So ist der Elektrodenkörper 15 auf einfache Art und Weise an der Elektrodeneinheit austauschbar, wenn dieser beispielsweise starken Verschleiß erlitten hat. Zusätzlich kann auch der Elektrodenkopf 14 ausgetauscht werden, um beispielsweise die Vorrichtung 1 an andere Fluide rasch anzupassen.

## Patentansprüche

1. Vorrichtung (1) zur elektrischen Desintegration von Zellverbänden, mit
einer Elektrodeneinheit (12), welche einen Elektrodenkopf (14) und einen Elektrodenkörper (15) aufweist; und
einer Kammer (4), die einen Einlass (6) zur Aufnahme von Zellverbände enthaltendem Fluid und einen Auslass (8) zur Abgabe des Fluids aufweist, wobei
die Kammer (4) eine Wand (5) aufweist, wobei die Wand (5) eine Mehrzahl voneinander beabstandeter, elektrisch leitfähiger Abschnitte (18) aufweist, die jeweils abwechselnd mit elektrisch isolierenden Abschnitten (20) entlang einer Zentralachse (A) der Kammer (4) angeordnet sind,
**dadurch gekennzeichnet, dass** der Elektrodenkörper (15) innerhalb der Kammer (4) und von der Wand (5) elektrisch isoliert angeordnet ist, und die elektrisch leitfähigen Abschnitte derart angeordnet sind, dass mittels Anlegen einer elektrischen Spannung zwischen dem Elektrodenkörper (15) und der Wand (5) eine Mehrzahl an Wirkfeldern erzeugbar ist.

2. Vorrichtung nach Anspruch 1,
**dadurch gekennzeichnet, dass** sich die Abschnitte (18, 20) jeweils ringförmig um die Kammer (4) herum erstrecken.

3. Vorrichtung nach einem der vorstehenden Ansprüche,
**dadurch gekennzeichnet, dass** die Wand (5) der Kammer (4) von einer inneren Oberfläche eines Rohres (2) gebildet wird.

4. Vorrichtung nach Anspruch 3,
**dadurch gekennzeichnet, dass** das Rohr (2) aus ringförmigen Abschnitten (18, 20) aus jeweils elektrisch leitfähigem und/oder elektrisch isolierendem Material gebildet ist.

5. Vorrichtung nach Anspruch 3,
**dadurch gekennzeichnet, dass** das Rohr (2) aus einem dünnwandigen elektrisch isolierenden Material gebildet ist und um das Rohr mehrere Manschetten (28) aus elektrisch leitendem Material angeordnet sind, die die elektrisch leitfähigen Abschnitte (18) der Wand (5) bilden.

6. Vorrichtung nach einem der vorstehenden Ansprüche,
**dadurch gekennzeichnet, dass** die elektrisch leitfähigen Abschnitte (18) jeweils von zwei elektrisch isolierenden Abschnitten (20) axial eingeschlossen sind.

7. Vorrichtung nach einem der vorstehenden Ansprüche,
**dadurch gekennzeichnet, dass** zwischen zwei benachbarten elektrisch leitfähigen Abschnitten (18) jeweils ein Mindestabstand (D) vorgesehen ist, welcher wenigstens beträgt: 150mm, 200mm, 250mm, 300mm oder 350mm.

8. Vorrichtung nach einem der vorstehenden Ansprüche,
**dadurch gekennzeichnet, dass** die elektrisch leitfähigen Abschnitte (18) eine axiale Ausdehnung (B) haben, die mindestens beträgt: 100mm, 150mm, 200mm oder 250mm.

9. Vorrichtung nach einem der vorstehenden Ansprüche,
**dadurch gekennzeichnet, dass** die elektrisch leitfähigen Abschnitte (18) geerdet sind.

10. Vorrichtung nach Anspruch 9,
**dadurch gekennzeichnet, dass** jeder elektrisch leitfähige Abschnitt (18) eine separate Erdung (30) aufweist.

11. Vorrichtung nach einem der vorstehenden Ansprüche,
**gekennzeichnet durch** ein Gehäuse (40), in welchem die Kammer (4) angeordnet ist und welches räumlich von der Wand (5) beabstandet und elektrisch isoliert ist.

12. Vorrichtung nach Anspruch 11,
**dadurch gekennzeichnet, dass** zwischen der Wand (19) und dem Gehäuse (40) jeweils ein Mindestabstand (M) vorgesehen ist, der beträgt: 100mm, 150mm, 200mm oder 250mm.

13. Vorrichtung nach einem der Ansprüche 11 oder 12,
**dadurch gekennzeichnet, dass** das Gehäuse (40) ein Isolationsmaterial aufweist oder aus diesem besteht wobei das Isolationsmaterial vorzugsweise ein Kunststoff, insbesondere PE und/oder ein Verbundmaterial, insbesondere ein Faserverbundwerkstoff aus Papier und/oder Glasfasern mit einem Phenol-Formaldehyd-Kunstharz und/oder einem Epoxidharz ist.

14. Vorrichtung (1) zur elektrischen Desintegration von Zellverbänden, mit
einer Kammer (4), die einen Einlass (6) zur Aufnahme von Zellverbände enthaltendem Fluid und einen Auslass (8) zur Abgabe des Fluids aufweist, wobei
die Kammer (4) eine Wand (5) aufweist und dazu angepasst ist, einen Elektrodenkörper (15) derart innerhalb der Kammer (4) aufzunehmen, dass dieser von der Wand (19) elektrisch isoliert angeordnet ist,
wobei die Wand (5) eine Mehrzahl voneinander beabstandeter, elektrisch leitfähiger Abschnitte (18) aufweist, die jeweils abwechselnd mit elektrisch isolierenden Abschnitten (20) entlang einer Zentralachse (A) der Kammer (4) angeordnet sind, und die derart angeordnet sind, dass mittels Anlegen einer elektrischen Spannung zwischen einem innerhalb der Kammer (4) angeordnetem Elektrodenkörper (15) und der Wand (5) eine Mehrzahl an Wirkfeldern erzeugbar ist.

## Claims

1. A device (1) for electrically disintegrating cell structures, having
an electrode unit (12) comprising an electrode head (14) and an electrode body (15), and having
a chamber (4) comprising an inlet (6) for receiving fluid comprising cell structures and an outlet (8) for discharging the fluid, the chamber (4) comprising a wall (5), the wall (5) comprising a plurality of electrically conductive segments (18) spaced apart from each other, disposed alternately with electrically insulating segments (20) along a central axis (A) of the chamber (4),
**characterized in that** the electrode body (15) is disposed within the chamber (4) and is electrically insulated from the wall (5), and the electrically conductive segments are disposed such that a plurality of effective fields can be generated by applying an electrical voltage between the electrode body (15) and the wall (5).

2. The device according to claim 1,
**characterized in that** the segments (18, 20) each extend annularly about the chamber (4).

3. The device according to any one of the preceding claims,
**characterized in that** the wall (5) of the chamber (4) is formed by an inner surface of a tube (2).

4. The device according to claim 3,
**characterized in that** the tube (2) is formed of annular segments (18, 20) each made of electrically conductive and/or electrically insulating material.

5. The device according to claim 3,
**characterized in that** the tube (2) is made of a thin-walled electrically insulating material and a plurality of cuffs (28) made of electrically conductive material are disposed about the tube and form the electrically conductive segments (18) of the wall (5).

6. The device according to any one of the preceding claims,
**characterized in that** the electrically conductive segments (18) are each axially enclosed by two electrically insulating segments (20).

7. The device according to any one of the preceding claims,
**characterized in that** a minimum spacing (D) is provided between adjacent electrically conductive segments (18) and comprises at least: 150mm, 200mm, 250mm, 300mm, or 350mm.

8. The device according to any one of the preceding claims,
**characterized in that** the electrically conductive segments (18) have an axial extent (B) of at least: 100mm, 150mm, 200mm, or 250mm.

9. The device according to any one of the preceding claims,
**characterized in that** the electrically conductive segments (18) are grounded.

10. The device according to claim 9,
**characterized in that** each electrically conductive segment (18) has a separate ground (30).

11. The device according to any one of the preceding claims,
**characterized by** a housing (40), spaced apart from the wall (5) and electrically insulated, in which the chamber (4) is disposed.

12. The device according to claim 11,
**characterized in that** a minimum spacing (M) is provided between the wall (19) and the housing (40) having the value of: 100mm, 150mm, 200mm, or 250mm.

13. The device according to any one of the claims 11 to 12,
**characterized in that** the housing (40) comprises an insulator material or is made thereof, wherein the insulator material is preferably a plastic, particularly PE and/or a composite material, particularly a fiber composite material made of paper and/or glass fibers having a phenol-formaldehyde synthetic resin and/or an epoxy resin.

14. A device (1) for electrically disintegrating cell structures, having
a chamber (4) comprising an inlet (6) for receiving fluid comprising cell structures and an outlet (8) for discharging the fluid,
the chamber (4) comprising a wall (5) and being adapted for receiving an electrode body (15) within the chamber (4), such that said electrode body is disposed electrically insulated from the wall (19),
the wall (5) comprising a plurality of electrically conductive segments (18) spaced apart from each other, disposed alternately with electrically insulating segments (20) along a central axis (A) of the chamber (4), and disposed such that a plurality of effective fields can be generated by applying an electrical voltage between an electrode body (15) disposed within the chamber (4) and the wall (5).

## Revendications

1. Dispositif (1) de désintégration électrique de structures cellulaires, comprenant une unité (12) d'électrode, qui a une tête (14) d'électrode et un corps (15) d'électrode et
une chambre (4), qui a une entrée (6) de réception d'un fluide comprenant des structures cellulaires et une sortie (8) de distribution du fluide, dans lequel
la chambre (4) a une paroi (5), la paroi (5) a une pluralité de parties (18) conductrices de l'électricité à distance les unes des autres, qui sont disposées chacune en alternance avec des parties (20) isolantes du point de vue électrique suivant un axe (A) central de la chambre (4),
**caractérisé en ce que** le corps (15) de l'électrode est disposé à l'intérieur de la chambre (4) et en étant isolé du point de vue électrique de la paroi (5) et les parties conductrices de l'électricité sont disposées de manière à pouvoir, en appliquant une tension électrique, produire une pluralité des champs actifs entre le corps (15) de l'électrode et la paroi (5).

2. Dispositif suivant la revendication 1,
**caractérisé en ce que** les parties (18, 20) s'étendent chacune annulairement autour de la chambre (4).

3. Dispositif suivant l'une des revendications précédentes,
**caractérisé en ce que** la paroi (5) de la chambre (4) est formée d'une surface intérieure d'un tube (2).

4. Dispositif suivant la revendication 3,
**caractérisé en ce que** le tube (2) est formé de parties (18, 20) annulaires en matériau respectivement conducteur du point de vue électrique et/ou isolant du point de vue électrique.

5. Dispositif suivant la revendication 3,
**caractérisé en ce que** le tube (2) est en un matériau à paroi mince isolant du point de vue électrique et, autour du tube, sont disposées plusieurs manchettes (28) en un matériau conducteur du point de vue électrique, qui forment les parties (18) conductrices du point de vue électrique de la paroi (5).

6. Dispositif suivant l'une des revendications précédentes,
**caractérisé en ce que** les parties (18) conductrices du point de vue électrique sont enfermées axialement chacune par deux parties (20) isolantes du point de vue électrique.

7. Dispositif suivant l'une des revendications précédentes,
**caractérisé en ce qu'**entre deux parties (18) voisines conductrices du point de vue électrique, est prévue respectivement une distance (D) minimum, qui est d'au moins 150 mm, 200 mm, 250 mm, 300 mm ou 350 mm.

8. Dispositif suivant l'une des revendications précédentes,
**caractérisé en ce que** les parties (18) conductrices du point de vue électrique ont une étendue axiale, qui est d'au moins 100 mm, 150 mm, 200 mm ou 250 mm.

9. Dispositif suivant l'une des revendications précédentes,
**caractérisé en ce que** les parties (18) conductrices du point de vue électrique sont mises à la terre.

10. Dispositif suivant la revendication 9,
**caractérisé en ce que** chaque partie (18) conductrice du point de vue électrique a une mise à la terre (30) distincte.

11. Dispositif suivant l'une des revendications précédentes,
**caractérisé par** un boîtier (40), dans lequel est disposée la chambre (4) et qui est à distance dans l'espace de la paroi (5) et qui est isolé du point de vue électrique.

12. Dispositif suivant la revendication 11,
**caractérisé en ce qu'**entre la paroi (19) et le boîtier (40), est prévue respectivement une distance (M) minimum, qui est de 100 mm, 150 mm, 200 mm ou 250 mm.

13. Dispositif suivant l'une des revendications 11 ou 12,
**caractérisé en ce que** le boîtier (40) a un matériau d'isolation ou en est constitué, le matériau d'isolation étant, de préférence, une matière plastique, notamment du PE, et/ou un matériau composite, notamment un matériau composite fibreux en papier et/ou en fibres de verre avec une résine phénol-formaldéhyde et/ou une résine époxyde.

14. Dispositif (1) de désintégration électrique de structures cellulaires, comprenant une chambre (4), qui a une entrée (6) de réception d'un fluide comprenant des structures cellulaires et une sortie (8) de distribution du fluide, dans lequel
la chambre (4) a une paroi (3) et est conçue pour recevoir un corps (15) d'électrode à l'intérieur de la chambre (4), de manière à ce que celui-ci soit isolé du point de vue électrique de la paroi (19),
dans lequel la paroi (5) a une pluralité de parties (18) à distance les unes des autres et conductrices du point de vue électrique, qui sont disposées chacune en alternance avec des parties (20) isolantes du point de vue électrique suivant un axe (A) central et qui sont disposées de manière à pouvoir, en appliquant une tension électrique entre un corps (15) de l'électrode disposé à l'intérieur de la chambre (4) et la paroi (5), produire une pluralité de champs actifs.
